# EUROPEAN PATENT SPECIFICATION

(11) **EP 3 858 274 B1**
(45) Date of publication and mention of the grant of the patent: **14.05.2025**
(21) Application number: 19867675.1
(22) Date of filing: 27.09.2019
(51) Int. Cl.: A61B 18/14, A61B 18/00

(54) **MEDICAL DEVICE**
MEDIZINISCHE VORRICHTUNG
DISPOSITIF MÉDICAL

(30) Priority: 27.09.2018 JP 2018181976
(43) Date of publication of application: 04.08.2021
(73) Proprietor: TERUMO Kabushiki Kaisha, Tokyo 151-0072 (JP)
(72) Inventor: ZHOU, Tuo, Ashigarakami-gun, Kanagawa 259-0151 (JP); OKUBO, Itaru, Ashigarakami-gun, Kanagawa 259-0151 (JP)
(74) Representative: Casalonga
(86) International application number: PCT/JP2019/038318
(87) International publication number: WO 2020/067491

(56) References cited:
- EP-A1- 3 219 278
- WO-A1-2014/083698
- WO-A1-2017/024055
- WO-A1-2017/223264
- JP-A- 2012 510 831
- JP-A- 2015 112 114
- US-B2- 9 937 340

## Description

### Technical Field

The present invention relates to a medical device inserted into a living body to perform ablation treatment on a biological tissue.

**In particular, the present invention relates to a medical device according to the preamble of claim 1,such as it is e.g. known from** WO 2017/223264 A1**,** EP 3 219 278 A1 **or** WO 2017/024055 A1**.**

### Background Art

As a medical device, a device that performs an irreversible electroporation (IRE) treatment is known. The irreversible electroporation has attracted attention since the treatment is non-thermal and can suppress damage to surrounding blood vessels or nerves. For example, a medical device is known in which a cancer that is less likely to be removed by surgery is treated by using the irreversible electroporation.

For atrial fibrillation caused by abnormal excitement appearing in a myocardial sleeve of a pulmonary vein wall, pulmonary vein isolation may be performed to destroy myocardial cells by ablating a joint portion between a pulmonary vein and a left atrium. In the pulmonary vein isolation, high frequency waves are generated from a distal end of an ablation catheter to cauterize and necrotize a myocardium in a dot shape. The ablation catheter is moved to cauterize an inflow portion of the pulmonary vein in a circumferential shape, and isolates the pulmonary vein.

For example, PTL 1 discloses a device in which a ring-shaped electrode is provided on an outer peripheral surface of an elongated tube body. A conductor for supplying a current to the electrode is spirally disposed inside the tube body.

### Citation List

### Patent Literature

PTL 1: US Patent No. 9,227,036

### Summary of Invention

### Technical Problem

It is desirable that a device inserted into a biological lumen has a reduced diameter so that the device can be inserted into a narrow biological lumen. However, according to the device disclosed in PTL 1 described above, an electrode is located outside a tube body with respect to a conductor in a radial direction. Therefore, it is difficult to reduce a diameter of the device inserted into a living body.

The present invention is made to solve the above-described problem, and an object thereof is to provide a medical device which can be inserted into a narrow biological lumen and can effectively ablate a wide range.

### Solution to Problem

In order to achieve the above-described object, there is provided a medical device according to claim 1. The dependent claims realte to advantaegous embodiments.

According to the medical device configured as described above, the electrode portion does not protrude outward in the radial direction, and can have a reduced diameter. Therefore, the medical device can be inserted into a narrow biological lumen, and a wide range can be effectively ablated by the electrode portion curved in the radial direction.

### Brief Description of Drawings

[Fig. 1] Fig. 1 is a front view illustrating a medical device according to a first embodiment.
[Fig. 2] Fig. 2 is a view illustrating the medical device. Fig. 2(A) is a sectional view taken along line A-A in Fig. 1, and Fig. 2(B) is a sectional view taken along line B-B in Fig. 1.
[Fig. 3] Fig. 3 is a sectional view illustrating a distal portion of the medical device.
[Fig. 4] Fig. 4 is a circumferential development view of a shaft portion which illustrates a conductor through an outer tube in a transparent view.
[Fig. 5] Fig. 5 is a sectional view illustrating the distal portion of the medical device in a state where an electrode portion is expanded.
[Fig. 6] Fig. 6 is front view illustrating a medical device according to a second embodiment.
[Fig. 7] Fig. 7 is a sectional view taken along line C-C in Fig. 6.
[Fig. 8] Fig. 8 is a sectional view illustrating a shaft portion.
[Fig. 9] Fig. 9 is a sectional view illustrating a distal portion of a medical device according to a third embodiment.
[Fig. 10] Fig. 10 is a sectional view taken along line D-D in Fig. 9.

### Description of Embodiments

Hereinafter, embodiments of the present invention will be described with reference to the drawings. Dimensions in the drawings are exaggerated and different from actual dimensions for convenience of description, in some cases. In addition, in the description herein and the drawings, the same reference numerals will be assigned to configuration elements having substantially the same functional configuration, and thus, repeated description will be omitted. In the description herein, a side where a device is inserted into a lumen will be referred to as a "distal side", and an operating hand-side will be referred to as a "proximal side".

### <First Embodiment>

A medical device 10 according to a first embodiment is percutaneously inserted into a biological lumen, comes into contact with a biological tissue of a target site, and applies an electric signal to perform irreversible electroporation. A target of the medical device 10 of the present embodiment is an electroporation treatment performed over an entire periphery of an entrance portion of a pulmonary vein in pulmonary vein isolation. However, the medical device 10 is also applicable to other treatments.

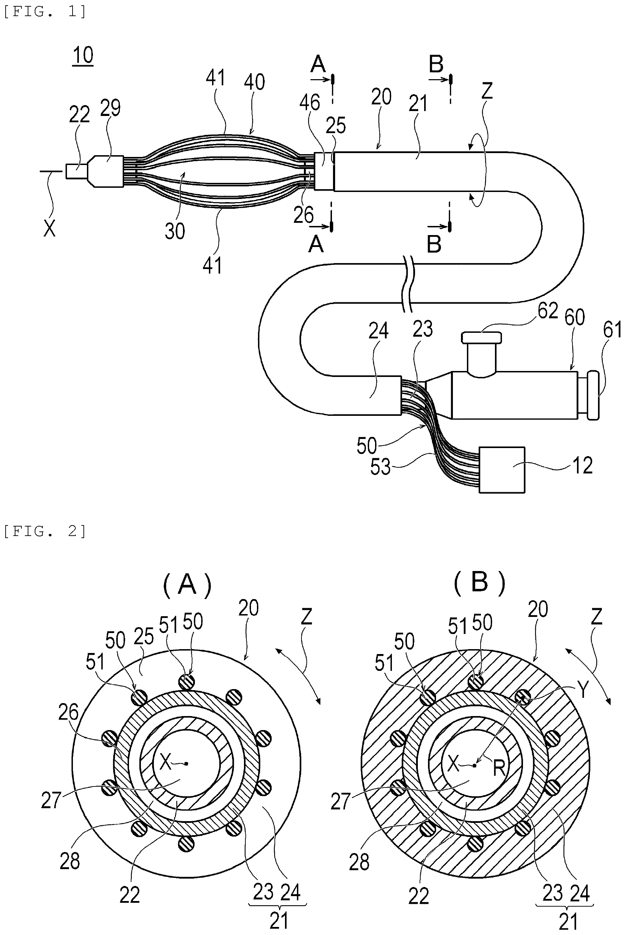

As illustrated in Figs. 1 to 3, the medical device 10 has an elongated shaft portion 20, a balloon 30 which is an expansion body provided in a distal portion of the shaft portion 20, and a hub 60 provided in a proximal portion of the shaft portion 20. Furthermore, the medical device 10 has a plurality of electrode portions 40 provided around the balloon 30 and a conductor 50 that transmits a current to the electrode portions 40.

The shaft portion 20 has a tubular outer tube 21 and an inner tube 22 disposed inside a first tube body 23. The outer tube 21 and the inner tube 22 are disposed coaxially with each other. The outer tube 21 and the inner tube 22 are relatively movable in an axial direction. The outer tube 21 has a tubular first tube body 23 and a second tube body 24 which covers an outer peripheral surface of the first tube body 23 and is fixed to the first tube body 23. The conductor 50 is disposed to be interposed between the first tube body 23 and the second tube body 24. The first tube body 23 and the second tube body 24 are disposed coaxially with each other. The first tube body 23 has a step portion 26 extending to a distal side further than a distal surface 25 of the second tube body 24. The step portion 26 has a circular tube shape. A proximal portion of the balloon 30 is fixed to an outer peripheral surface of the step portion 26. In addition, a connection section 54 between the electrode portion 40 and the conductor 50 may be disposed on the outer peripheral surface of the step portion 26. In addition, the conductors 50 are likely to be equally disposed in a circumferential direction Z since the conductors 50 are disposed on an outer surface of the first tube body 23 disposed inside the second tube body 24.

A guide wire lumen 27 extending along a length direction is formed inside the inner tube 22. A guide wire can be inserted into the guide wire lumen 27. An inflation lumen 28 is formed inside the outer tube 21 and outside the inner tube 22. An inflation fluid for inflating the balloon 30 can flow through the inflation lumen 28. The inflation fluid may be gas or a liquid. For example, it is possible to use gas such as helium gas, CO₂ gas, O₂ gas, and laughter gas, or a liquid such as a physiological salt solution, a contrast agent, and a mixture thereof.

The inner tube 22 further extends to a distal side than a distal end of the first tube body 23. A distal portion of the balloon 30 is fixed to an outer peripheral surface of the inner tube 22 on a distal side from the first tube body 23. In the inner tube 22, a fixing portion 29 for fixing a distal portion of the electrode portion 40 is fixed to an outer peripheral surface on a distal side from a position where the balloon 30 is fixed.

An outer diameter of the shaft portion 20 is not particularly limited. However, it is preferable that the outer diameter is minimally invasive and is not excessively large to satisfy compatibility with a general sheath or a guiding catheter to be inserted. For example, the outer diameter is 4.0 mm or smaller, and is preferably 2.9 mm or smaller.

It is preferable that a material for forming the first tube body 23, the second tube body 24, and the inner tube 22 has flexibility to some degrees. In addition, it is preferable that the material for forming of the first tube body 23, the second tube body 24, and the inner tube 22 has an insulation property. Examples of the material include polyolefin such as polyethylene, polypropylene, polybutene, ethylene-propylene copolymer, ethylene-vinyl acetate copolymer, ionomer, and a mixture of two or more types thereof, and soft polyvinyl chloride resin, polyamide, polyamide elastomer, polyimide, polyester, polyester elastomer, polyurethane, fluororesin such as polytetrafluoroethylene, silicone rubber, and latex rubber.

The balloon 30 is flexible and deformable. A shape of the balloon 30 is not particularly limited. However, for example, the shape may be a cylinder, an ellipsoid, or a trapezoid. The distal portion of the balloon 30 is fixed to the outer peripheral surface of the distal portion of the inner tube 22. The proximal portion of the balloon 30 is fixed to the outer peripheral surface of the distal portion of the first tube body 23. It is preferable that the balloon 30 has a thin film shape and flexibility. In addition, the balloon 30 needs to be strong enough to reliably spread the electrode portion 40. As a material for forming the balloon 30, the above-described materials for forming the shaft portion 20 can be used. Alternatively, it is possible to use other materials (for example, various elastomer materials such as hydrogenated styrene-based thermoplastic elastomer (SEBS)).

The conductor 50 has a linear shape. As illustrated in Figs. 1 to 4, the conductor 50 has a buried portion 52 buried inside the outer tube 21, a protruding portion 51 protruding in a distal direction from the distal surface 25 of the outer tube 21, and a lead-out portion 53 which is led out from the outer tube 21 on the proximal side. The buried portion 52 is interposed between the first tube body 23 and the second tube body 24. The buried portion 52 is located outside an inner peripheral surface of the outer tube 21, and is located inside the outer peripheral surface. For example, a wire rod serving as the conductor 50 is wound around the outer peripheral surface of the first tube body 23 and the second tube body 24 is extruded outward. In this manner, the buried portion 52 is buried in the outer tube 21. It is preferable that the buried portion 52 is covered by a material of the outer tube 21 formed of an insulation material without any gap. In this manner, it is possible to reliably suppress electrical short-circuit of the buried portions 52 which may come into contact with each other. The number of the conductors 50 is equal to or more than the number of the electrode portions 40. In this manner, the conductor 50 can transmit an independent current to all of the electrode portions 40. In the present embodiment, the number of the conductors 50 is the same as the number of the electrode portions 40 (for example, 10). The plurality of conductors 50 are formed in a structure which is spirally wound multiple times. The plurality of conductors 50 are disposed away from each other at an equal interval. Therefore, the plurality of the conductors 50 can transmit the independent current without any electrical short-circuit. A separation distance between the conductors 50 is preferably set in accordance with a supplied electric signal so that the electrical short-circuit does not occur. For example, when an electric signal of 1,000 V is applied, the separation distance between the conductors 50 is preferably 10 µm or longer. In this manner, it is possible to improve electrical safety of the medical device 10.

When a radius (distance) from an axis X of the shaft portion 20 to a central axis Y of the conductor 50 is defined as R, the number of the conductors 50 is defined as N, and a tilting angle of the conductor 50 with respect to a cross section orthogonal to the axis X of the shaft portion 20 is defined as θ, a distance D between the central axes Y of the adjacent conductors 50 is 2πR/N·sinθ.

For example, the radius R is 0.2 to 2.6 mm. For example, the number N is 2 to 20. For example, the tilting angle θ is 1 to 45 degrees. For example, the distance D is 0.02 to 8 mm.

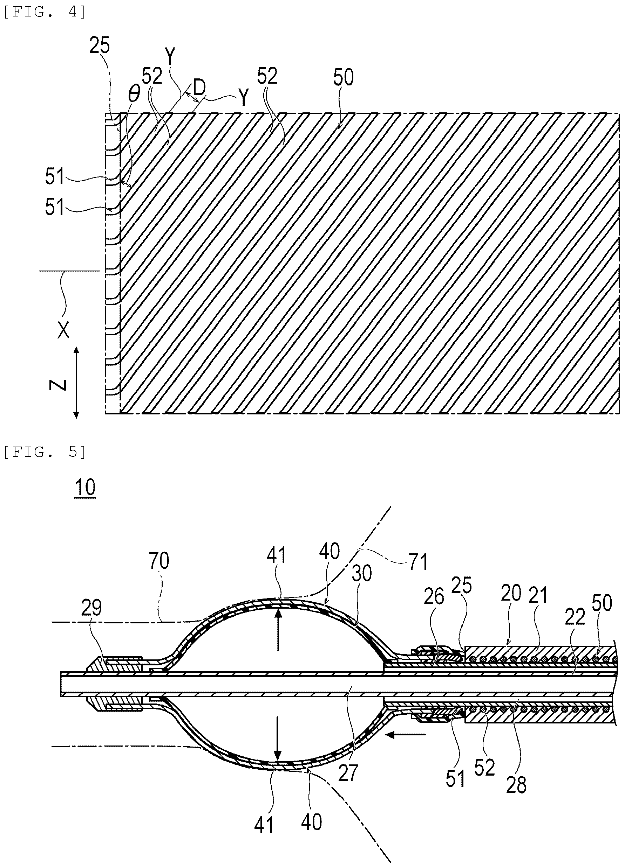

The protruding portion 51 is substantially parallel to the axis X, and is connected to the proximal portion of the electrode portion 40. At least a portion of the protruding portion 51 is perpendicular to the circumferential direction Z of the shaft portion 20. That is, at least a portion of the protruding portion 51 is parallel to the axis X of the shaft portion, when a development view of the shaft portion 20 developed in the circumferential direction Z is viewed from the outside in the radial direction of the shaft portion 20 (refer to Fig. 4). Therefore, when the protruding portion 51 protrudes in the distal direction from the buried portion 52 spirally wound inside the outer tube 21 through the distal surface 25, the angle is changed, and the protruding portion is substantially parallel to the axis X. The protruding portion 51 may not be parallel to the axis X. For example, the protruding portion 51 may be tilted in the distal direction to be closer to the axis X of the shaft portion 20. Even in this case, the protruding portion 51 can be perpendicular to the circumferential direction Z. The distal end of the protruding portion 51 is located on the proximal side from the distal end of the step portion 26. Therefore, the proximal portion of the protruding portion 51 and the electrode portion 40 is stably disposed on an outer surface of the step portion 26.

The lead-out portion 53 is drawn out in a proximal direction from the proximal portion of the second tube body 24, and is connected to a power source unit 12 provided outside the shaft portion 20. The power source unit 12 can supply electricity to the electrode portion 40.

A material for forming the conductor 50 is preferably highly conductive. For example, copper, gold, platinum, silver, aluminum, alloy, or carbon fiber may be used. As the conductor 50, a known lead wire can be used.

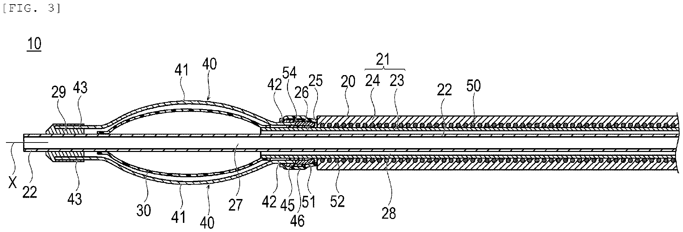

As illustrated in Figs. 2 and 3, each of the electrode portions 40 is located on an outer peripheral side of the balloon 30, and is not fixed to the balloon 30. The electrode portion 40 may be fixed to the balloon 30. The electrode portion 40 is formed of a wire rod which is conductive and flexible. The plurality of electrode portions 40 are disposed to be aligned in the circumferential direction Z of the balloon 30 on the outer peripheral side of the balloon 30. Each of the electrode portions 40 extends in the length direction of the shaft portion 20. The electrode portion 40 includes a curved portion 41 that can be curved in the radial direction of the shaft portion 20. The electrode portion 40 has an electrode proximal portion 42 on the proximal side from the balloon 30 and an electrode distal portion 43 on the distal side from the balloon 30.

A proximal end of the electrode proximal portion 42 is in contact with and joined to a distal end of the protruding portion 51 of the conductor 50. In this manner, the electrode portion 40 is electrically connected to the conductor 50. The connection section 54 between the electrode proximal portion 42 and the protruding portion 51 which are provided in each of the electrode portions 40 is covered by an insulation tube 45 formed of an insulation material. A joining method is not limited as long as the method enables electrical conduction. For example, soldering, laser fusion, welding using various metal braces, bonding using a conductive adhesive, or mechanical interlocking using a chuck may be used. The plurality of connection sections 54, the insulation tube 45, and the protruding portion 51 which are aligned on the outer peripheral surface of the step portion 26 are collectively covered by a single protective tube 46 formed of an insulation material. Therefore, the connection section 54 and the protruding portion 51 are covered by the insulation tube 45 and/or the protective tube 46 on the distal side from the distal surface 25, and are not exposed outward. In this manner, it is possible to improve electrical safety of the medical device 10. As a material for forming the insulation tube 45 and the protective tube 46, the above-described materials for forming the shaft portion 20 can be used.

The electrode distal portion 43 is fixed to the fixing portion 29 provided on the distal side from the balloon 30 of the inner tube 22. A cross-sectional shape orthogonal to the length direction of each of the electrode portions 40 is rectangular. That is, the cross-sectional shape orthogonal to the length direction of the electrode portion 40 is a shape formed so that in at least a portion of the electrode portion 40, the length along the circumferential direction Z of the balloon 30 is longer than the length along the radial direction of the balloon 30. Therefore, a long side of the cross section extends along the circumferential direction Z of the balloon 30. In this manner, the plurality of electrode portions 40 aligned in the circumferential direction Z of the balloon 30 are likely to be bent in the radial direction of the balloon 30, and are less likely to deform in a direction in which the electrode portions 40 are closer to each other. Therefore, it is possible to suppress electrical short-circuit or entanglement between the electrode portions 40. A cross-sectional shape of the electrode portion 40 is not limited to a rectangle, and for example, may be a circle, a semicircle, an ellipse, or a square.

As a material for forming the electrode portion 40, for example, superelastic metal represented by a Ni-Ti alloy can be preferably used. However, the electrode portion 40 may be formed of a conductive material other than the above-described material. For example, the electrode portion 40 may be conductive rubber. Furthermore, the electrode portion 40 may be formed of a flexible printed circuit board (FPC) .

In the electrode portion 40, the outer surface other than the curved portion 41 is coated with an insulation material. The insulation material is not electrically conductive. The insulation material may be provided on a side where the outer surface of the electrode portion 40 is not in contact with the biological tissue, that is, a side that faces the balloon 30.

In the present embodiment, 10 electrode portions 40 are equally provided in the circumferential direction Z. However, the number of the electrode portions 40 may be larger or smaller than 10. An electric signal is applied between the adjacent electrode portions 40. However, an electrode (alternatively, a counter electrode) may be disposed outside a body, and the electric signal may be applied between the electrode (alternatively, the counter electrode) outside the body and the electrode portion 40.

As illustrated in Fig. 1, the proximal portion of the inner tube 22 is interlocked with the hub 60. The first tube body 23 located outside the inner tube 22 and movable in the axial direction with respect to the inner tube 22 is interlocked with the hub 60 to be slidable. The hub 60 has a first port 61 having an opening communicating with the guide wire lumen 27 and a second port 62 having an opening communicating with the inflation lumen 28.

Next, an unclaimed treatment method using the medical device 10 will be described. First, an introducer (not illustrated) percutaneously punctures the blood vessel. Next, after a guide wire (not illustrated) is inserted into a guiding catheter (not illustrated), the guiding catheter is inserted into the introducer. Next, the guide wire is protruded to the distal side, and thereafter, a distal portion of the guiding catheter is inserted into the blood vessel through a distal portion opening of the introducer. Thereafter, while the guide wire is moved ahead, the guiding catheter is gradually pushed to a target site. An operator forms a through-hole in an atrial septum by penetrating a predetermined puncture device from a right atrium side toward a left atrium side. For example, as the puncture device, it is possible to use a device such as a wire having a sharp distal end. The puncture device can be delivered via the guiding catheter. In addition, for example, the puncture device can be delivered into the atrial septum instead of the guide wire after the guide wire is removed from the guiding catheter. A specific structure of the puncture device used for penetrating the atrial septum, and a specific procedure for forming the through-hole is not particularly limited. After the through-hole is formed, the operator uses a dilator to widen the through-hole. Next, the operator causes the guiding catheter to pass through the through-hole, and uses the guide wire to push the guiding catheter forward to the target site (for example, vicinity of the pulmonary vein).

Next, an end of the guide wire is inserted into a distal opening portion of the guide wire lumen 27 of the shaft portion 20, and the guide wire is pulled out from the first port 61 of the hub 60. Next, the medical device 10 is inserted from the distal portion into the guiding catheter inserted into the blood vessel, and the medical device is pushed forward along the guide wire. At this time, a ring catheter provided with an electrode may be used instead of the guide wire.

As illustrated in Fig. 5, after the electrode portion 40 is inserted into an entrance of a pulmonary vein 70 which is a target position, the inflation fluid is supplied into the balloon 30 via the second port 62 and the inflation lumen 28. In this manner, the balloon 30 is inflated, and the electrode portion 40 pushed by the balloon 30 is expanded in the radial direction. At this time, the outer tube 21 moves to the distal side with respect to the inner tube 22, and the proximal portion of the electrode portion 40 moves to the distal side. In this manner, the electrode portion 40 can deform while following the inflation of the balloon 30. Therefore, the curved portion 41 located in a central portion of the electrode portion 40 is pushed against a biological wall 71 by the balloon 30. The electrode portion 40 can deform to fit a shape of the biological tissue. Therefore, the electrode portion 40 can be brought into close contact with the biological tissue, and an electric signal is likely to be applied. In this state, the electric signal is applied from the power source unit 12 to the electrode portion 40 via the conductor 50.

A pulsed electric signal is first applied from the power source unit 12 to a pair of electrode portions 40 and 40 adjacent to each other in the circumferential direction Z. In this manner, a current flows between the pair of electrode portions 40 and 40 adjacent to each other in the circumferential direction Z. Next, the pulsed electric signal is applied to the other pair of electrode portions 40 and 40 adjacent to each other in the circumferential direction Z. The electric signals are sequentially applied to all pairs of the electrode portions 40 and 40 adjacent to each other in the circumferential direction Z. An example of the applied electric signal will be described below. Electric field intensity applied by the power source unit 12 is 1,500 V/cm, and a pulse width of the electric signal is 100 µsec. The electric signals are repeatedly applied to all pairs of the electrode portions 40 adjacent to each other in the circumferential direction Z, 60 to 180 times in a cycle of once every 2 seconds, depending on a refractory period of a ventricular muscle. In this manner, cells in the entrance of the pulmonary vein are necrotized over the entire periphery. The electric signal may be applied between the plurality of electrode portions 40 that are not adjacent to each other, or the electric signal may be applied from the electrode portion 40 to a counter electrode attached to a body surface.

When the electric signal is completely applied, the balloon 30 is deflated. In this manner, the electrode portion 40 is contracted in the radial direction due to a self-restoring force. At this time, the outer tube 21 moves to the proximal side with respect to the inner tube 22, and the proximal portion of the electrode portion 40 moves to the proximal side. In this manner, the electrode portion 40 can deform while following the deflation of the balloon 30. Thereafter, all instruments inserted into the blood vessels are removed to complete the procedure. When the electrode portion 40 is formed of a material other than the superelastic alloy such as the Ni-Ti alloy, it is preferable to perform an operation for contracting the electrode portion 40 in the radial direction by pushing the inner tube 22 to the distal side (or by pulling the outer tube 21 to the proximal side) .

As described above, the medical device 10 according to the first embodiment includes the elongated shaft portion 20, the plurality of electrically independent electrode portions 40 disposed on the distal portion of the shaft portion 20, extending along the length direction of the shaft portion 20, and deformable in the radial direction of the shaft portion 20, and the plurality of electrically independent conductors 50 including the buried portion 52 buried in the shaft portion 20, and allowing the current to flow to the electrode portions 40. At least one of the conductors 50 has the protruding portion 51 protruding from the distal surface 25 of the shaft portion 20, and connected to the electrode portion 40.

In the medical device 10 configured as described above, the buried portion 52 on the proximal side of the plurality of conductors 50 is buried in the shaft portion 20. The protruding portion 51 on the distal side connected to the electrode portion 40 protrudes from the distal surface 25 of the shaft portion 20. Therefore, the electrode portion 40 can be located inside the outer diameter of the shaft portion 40. Accordingly, the electrode portion 40 does not protrude outward in the radial direction, and can have a reduced diameter. Therefore, the medical device 10 can be inserted into a narrow biological lumen, and a wide range can be effectively ablated by the electrode portion 40 curved in the radial direction. The electrode portion 40 may be located at a position the same as that of the outer peripheral surface of the shaft portion 40. Furthermore, the conductor 50 is buried in the shaft portion 20. Accordingly, bending rigidity of the shaft portion 20 is increased, and kink resistance is improved. For example, when the electrode portion 40 is brought into contact with the vicinity of the entrance of the pulmonary vein 70, the shaft portion 20 cannot move in the radial direction in a puncture site of the atrial septum (for example, an oval fossa). Accordingly, the shaft portion 20 is curved. At this time, since the bending rigidity of the shaft portion 20 is increased, kink of the shaft portion 20 can be suppressed.

In addition, each of the plurality of conductors 40 has the protruding portion 51, and each of the protruding portions 51 is connected to the different electrode portion 40. In this manner, the independent electric signal can be applied to each of the plurality of electrode portions 40.

In addition, the protruding portion 51 is connected to the electrode portion 40 located on the distal side of the protruding portion 51 in the axial direction. In this manner, the electrode portion 40 does not protrude outward in the radial direction, and the medical device 10 can easily have the reduced diameter.

In addition, at least a portion of the protruding portion 51 is perpendicular to the circumferential direction Z of the shaft portion 20. In this manner, at least one of the electrode portion 40 and the protruding portion 51 can be accurately disposed at a suitable position in the circumferential direction Z of the shaft portion 20.

In addition, at least a portion of the protruding portion 51 is parallel to the axis X of the shaft portion 20, and at least a portion of the buried portion 52 has a spiral shape wound around the axis X of the shaft portion 20. In this manner, the bending rigidity of the shaft portion 20 is not biased by a bending direction, and the shaft portion 20 having uniform quality can be formed.

In addition, the plurality of electrode portions 40 are equally disposed in the circumferential direction Z of the shaft portion 20. In this manner, the electrode portion 40 can evenly ablate the target site.

In addition, when the radius from the axis X of the shaft portion 20 to the conductor 50 is defined as R, the number of the conductors 50 is defined as N, and the tilting angle of the conductor 50 with respect to the cross section orthogonal to the axis X of the shaft portion 20 is defined as θ, the distance D between central axes Y of the conductors 50 adjacent to each other is 2πR/N·sinθ. In this manner, the conductors 50 can be equally disposed in the circumferential direction Z of the shaft portion 20. Therefore, when the electrode portions 40 are equally disposed in the circumferential direction Z, the electrode portions 40 are easily disposed so that the position of the conductor 50 is aligned with the electrode portion 40.

In addition, the shaft portion 20 has the step portion 26 protruding to the distal side of the distal surface 25 from a position inside the distal surface 25 in the radial direction. In this manner, the protruding portion 51 of the conductor 50 can be supported by the step portion 26, and disconnection of the conductor 50 can be suppressed. In the present embodiment, the outer tube 21 of the shaft portion 20 has the first outer tube 23 including the step portion 26, and the second tube body 24 covering the outer peripheral surface of the first tube body 23 and fixed to the first tube body 23. Accordingly, it is easy to form the step portion 26 from a position inside the distal surface 25 in the radial direction.

Also, the medical device 10 has the expansion body (for example, the balloon 30) located between the shaft portion 20 and the electrode portion 40, and inflatable outward in the radial direction of the shaft portion 20. In this manner, the medical device 10 can bring the electrode portion 40 into close contact with the biological tissue by inflating the expansion body.

### <Second Embodiment>

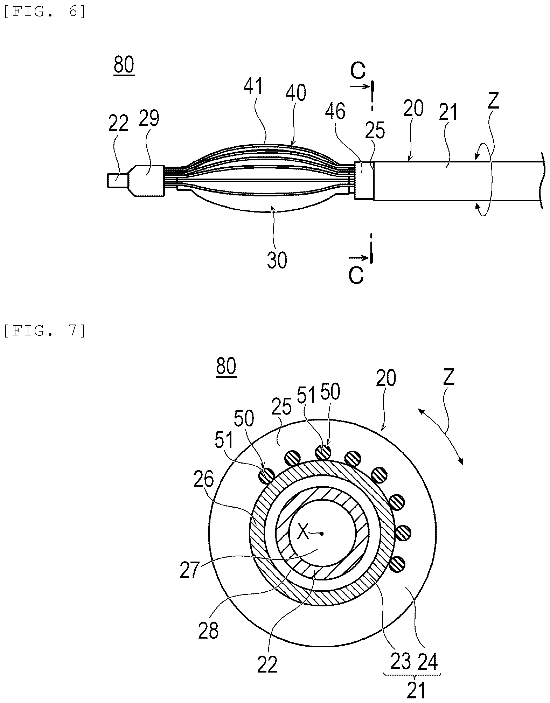

A medical device 80 according to a second embodiment is different from that according to the first embodiment only in the following point. As illustrated in Figs. 6 to 8, the electrode portion 40 and the conductor 50 are provided to be biased in the circumferential direction Z of the shaft portion 20.

The plurality of the conductors 50 have the protruding portion 51 protruding from the distal surface 25 of the shaft portion 20 in the distal direction at a position biased in the circumferential direction Z of the shaft portion 20. Therefore, the plurality of conductors 50 inside the shaft portion 20 collectively form one group, and are disposed in a spiral shape while a gap 81 is interposed therebetween in a collective state. The protruding portion 51 and the electrode proximal portion 42 of the plurality of electrode portions 40 are located on the distal side of the plurality of the protruding portions 51. Therefore, it is easy to electrically connect the electrode proximal portion 42 to the protruding portion 51.

As described above, in the medical device 80 according to the second embodiment, the plurality of electrode portions 40 are disposed to be biased to a portion of the shaft portion 20 in the circumferential direction Z. In this manner, in the medical device 80, only a specific site in the circumferential direction Z can be intentionally ablated by the electrode portion 40. Therefore, in the medical device 80, it is possible to easily adjust the electrode portion 40 so that only the specific site is ablated and other sites are not ablated. A biasing method of the protruding portion 51 is not particularly limited. Therefore, the protruding portion 51 and the electrode portion 40 may be provided to be biased at a plurality of locations in the circumferential direction Z of the shaft portion 20. In addition, the distance between the adjacent protruding portions 51 and the distance between the adjacent electrode portions 40 may not be uniform.

### <Third Embodiment>

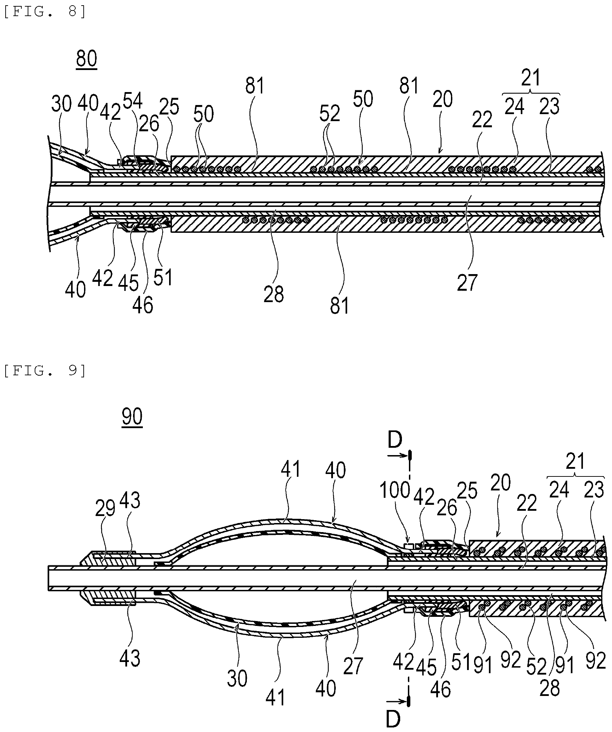

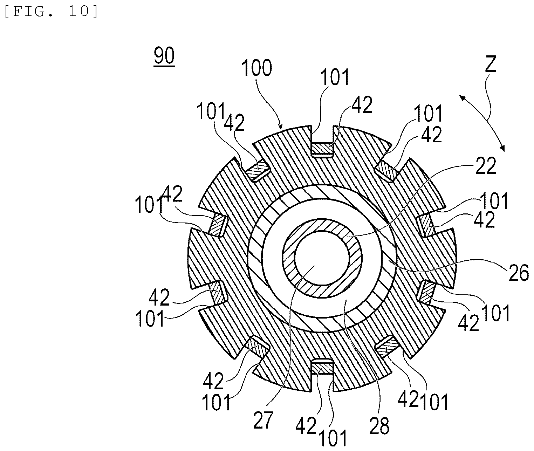

A medical device 90 according to a third embodiment is different from that according to the first embodiment only in that the conductor 50 is braided, and has a support body 100 as illustrated in Figs. 9 and 10.

A plurality of braided wire rods 91 are disposed inside the shaft portion 20. A portion of the wire rods 91 is used as the conductor 50. All of the wire rods 91 may be used as the conductors 50. The wire rod 91 used as the conductor 50 is formed of a conductive material, and a surface thereof is coated with the insulation layer 92. The wire rod 91 which is not used as the conductor 50 may be formed of the conductive material, or may not be formed of the conductive material. When the wire rod 91 which is not used as the conductor 50 is not formed of the conductive material, it is possible to suppress the electrical short-circuit with the wire rod 91 which is used as the conductor 50. In the wire rod 91 which is not used as the conductor 50, the surface may be coated with or may not be coated with the insulation layer 92. The support body 100 that supports the electrode proximal portion 42 of the plurality of electrode portions 40 is fixed to an outer surface of the step portion 26 of the shaft portion 20.

The support body 100 has a tubular shape, and a plurality of housing portions 101 that house the electrode proximal portions 42 of the electrode portions 40 are formed on the outer peripheral surface. The support body 100 is located away from the distal surface 25 in the distal direction. The support body 100 may not be located away from the distal surface 25 in the distal direction. The housing portion 101 is a groove extending in the length direction of the shaft portion 20. The plurality of housing portions 101 are formed to be equally aligned in the circumferential direction Z of the support body 100. Each of the housing portions 101 houses one of the electrode proximal portions 42. The electrode proximal portion 42 is fixed to the support body 100 by using an adhesive in a state of being housed in the support body 100. The support body 100 may house the protruding portion 51 of the conductor 50 instead of the electrode proximal portion 42. Alternatively, the support body 100 may house both the electrode proximal portion 42 and the protruding portion 51. In addition, the support body 100 may be provided on the outer peripheral surface of the inner tube 22 located on the distal side from the balloon 30 to house the electrode distal portion 43 located on the distal side of the electrode portion 40.

As described above, the medical device 90 according to the third embodiment further has the annular support body 100 disposed on the outer peripheral surface of the shaft portion 20, and the support body 100 has the housing portion 100 that houses at least one of the electrode portion 40 and the protruding portion 51. In this manner, at least one of the electrode portion 40 and the protruding portion 51 can be accurately disposed at a suitable position in the circumferential direction Z of the shaft portion 20.

In addition, the support body 100 is located away from the distal surface 25 in the distal direction. In this manner, the protruding portion 51 protruding from the distal surface 25 has a suitable posture in the gap between the support body 100 and the distal surface 25, and is disposed at a suitable position of the support body 100.

In addition, the conductor 50 is at least a portion of the plurality of wire rods 91 braided in a spiral shape, and the surface is covered with the insulation layer 92. In this manner, the conductor 50 can use the braided wire rod 91, and even when the conductors 50 come into contact with each other due to the braiding, the insulation layer 92 can suppress the electrical short-circuit.

The present invention is not limited to the above-described embodiments, and various modifications can be made by those skilled in the art within the scope of the present invention. For example, a configuration has been described in which the medical device 10 according to the above-described embodiments is used for treating the pulmonary vein. However, the medical device 10 may be used for treating other sites, for example, such as a renal artery, an ascending vena cava, and a ventricle.

In addition, the conductor 50 which transmits the current to the electrode portion 40 may not have a spiral shape, or may not be a portion of the braided wire rod 91. For example, the conductor 50 may be the wire rod linearly extending along the axis X of the shaft portion 20. In addition, the conductor 50 may be the wire rod that is curved or bent.

In addition, the expansion body of the medical device may not be the balloon 30. In addition, the medical device may not have the expansion body that presses the electrode portion 40 outward in the radial direction. For example, the medical device can expand the electrode portion 40 in the radial direction of the shaft portion 20 by moving the outer tube 21 with respect to the inner tube 22 in the distal direction without being provided with the balloon 30. In addition, when the electrode portion 40 or a portion of the conductor 50 is provided with an extendable portion, the outer tube 21 and the inner tube 22 may not be relatively movable in the axial direction. In this case, even when the balloon 30 is inflated and the electrode portion 40 is curved outward in the radial direction, the extendable portion extends. Accordingly, the outer tube 21 and the inner tube 22 do not need to relatively move. Alternatively, the fixing portion 29 may be slidable in the axial direction with respect to the outer peripheral surface of the inner tube 22. In addition, the conductor may be buried in the inner tube 22 instead of the outer tube 21. In this case, the conductor is electrically connected to the distal portion of the electrode portion 40.

This application is based on Japanese Patent Application No. 2018-181976 filed on September 27, 2018.

### Reference Signs List

- 10, 80, 90: medical device
- 20: shaft portion
- 21: outer tube
- 22: inner tube
- 23: first tube body
- 24: second tube body
- 25: distal surface
- 26: step portion
- 30: balloon (expansion body)
- 40: electrode portion
- 41: curved portion
- 50: conductor
- 51: protruding portion
- 52: buried portion
- 53: connection section
- 91: wire rod
- 92: insulation layer
- 100: support body
- 101: housing portion
- D: distance between central axes of conductors adjacent to each other
- N: number of conductors
- R: radius from axis of shaft portion to central axis of conductor
- X: axis
- Y: central axis of conductor
- Z: circumferential direction
- θ: tilting angle of conductor

## Claims

1. A medical device (10, 80, 90) comprising:
an elongated shaft portion (20);
a plurality of electrically independent electrode portions (40) disposed on a distal side of the shaft portion (20), extending along a length direction of the shaft portion (20), and deformable in a radial direction of the shaft portion (20); and
a plurality of electrically independent conductors (50) including a buried portion (52) buried in the shaft portion (20), and allowing a current to flow to the electrode portions (40),
**characterised in that**
each of the plurality of conductors (50) has a protruding portion (51) protruding from a distal surface (25) of the shaft portion (20) and connected to the electrode portion (40) and each of the protruding portions (51) is connected to each of the electrode portions (40) different from each other,
wherein
each of the protruding portions (51) is parallel to an axis (X) of the shaft portion (20), and at least a portion of the buried portion (52) has a spiral shape wound around the axis of the shaft portion.

2. The medical device (10, 80, 90) according to Claim 1,
wherein the plurality of electrode portions (40) are equally disposed in a circumferential direction (Z) of the shaft portion (20).

3. The medical device (10, 80, 90) according to Claims 1 or 2,
wherein when a radius from an axis (X) of the shaft portion (20) to the conductor (50) is defined as R, the number of the conductors (50) is defined as N, and a tilting angle of the conductor (50) with respect to a cross section orthogonal to the axis (X) of the shaft portion (20) is defined as θ, a distance between central axes of the conductors (50) adjacent to each other is 2πR/N·sinθ.

4. The medical device (10, 80, 90) according to Claim 1,
wherein the plurality of electrode portions (40) are disposed to be biased to a portion of the shaft portion (20) in a circumferential direction (Z).

5. The medical device (10, 80, 90) according to any one of Claims 1 to 4, further comprising:
an annular support body disposed on an outer peripheral surface of the shaft portion (20),
wherein the support body has a housing portion (101) that houses at least one of the electrode portion (40) and the protruding portion (51).

6. The medical device (10, 80, 90) according to any one of Claims 1 to 5,
wherein the shaft portion (20) has a step portion (26) protruding from a position inside the distal surface (25) in the radial direction to a distal side of the distal surface (25).

7. The medical device (10, 80, 90) according to any one of Claims 1 to 6,
wherein the conductor (50) is at least a portion of a plurality of spirally braided wire rods (91), and a surface of which is coated with an insulation material.

8. The medical device (10, 80, 90) according to any one of Claims 1 to 7, further comprising:
an expansion body (30) located between the shaft portion (20) and the electrode portion (40), and inflatable outward in the radial direction of the shaft portion (20).

## Patentansprüche

1. Medizinische Vorrichtung (10, 80, 90), umfassend:
einen langgestreckten Schaftabschnitt (20);
eine Vielzahl von elektrisch unabhängigen Elektrodenabschnitten (40), die an einer distalen Seite des Schaftabschnitts (20) angeordnet sind, sich entlang einer Längsrichtung des Schaftabschnitts (20) erstrecken und in einer radialen Richtung des Schaftabschnitts (20) verformbar sind; und
eine Vielzahl von elektrisch unabhängigen Leitungen (50), die einen verborgenen Abschnitt (52) umfassen, der in dem Schaftabschnitt (20) verborgen ist und es einem Strom ermöglicht, zu den Elektrodenabschnitten (40) zu fließen,
**dadurch gekennzeichnet, dass**
jede der Vielzahl von Leitungen (50) einen hervorstehenden Abschnitt (51) aufweist, der von einer distalen Oberfläche (25) des Schaftabschnitts (20) hervorsteht und mit dem Elektrodenabschnitt (40) verbunden ist, und
jeder der hervorstehenden Abschnitte (51) mit jedem der Elektrodenabschnitte (40) unterschiedlich voneinander verbunden ist,
wobei jeder der hervorstehenden Abschnitte (51) parallel zu einer Achse (X) des Schaftabschnitts (20) ist und mindestens ein Abschnitt des verborgenen Abschnitts (52) eine um die Achse des Schaftabschnitts gewundene Spiralform aufweist.

2. Medizinische Vorrichtung (10, 80, 90) nach Anspruch 1,
wobei die Vielzahl von Elektrodenabschnitten (40) gleichmäßig in einer Umfangsrichtung (Z) des Schaftabschnitts (20) angeordnet sind.

3. Medizinische Vorrichtung (10, 80, 90) nach den Ansprüchen 1 oder 2,
wobei, wenn ein Radius von einer Achse (X) des Schaftabschnitts (20) zu der Leitung (50) als R definiert ist, die Anzahl der Leitungen (50) als N definiert ist und ein Neigungswinkel der Leitung (50) in Bezug auf einen Querschnitt orthogonal zu der Achse (X) des Schaftabschnitts (20) als θ definiert ist, ein Abstand zwischen Mittelachsen der Leitungen (50), die nebeneinander sind, 2πR/N·sinθ beträgt.

4. Medizinische Vorrichtung (10, 80, 90) nach Anspruch 1,
wobei die Vielzahl von Elektrodenabschnitten (40) so angeordnet sind, dass sie in einer Umfangsrichtung (Z) gegen einen Abschnitt des Schaftabschnitts (20) vorgespannt sind.

5. Medizinische Vorrichtung (10, 80, 90) nach einem der Ansprüche 1 bis 4, ferner umfassend:
einen ringförmigen Stützkörper, der an einer äußeren Umfangsfläche des Schaftabschnitts (20) angeordnet ist,
wobei der Stützkörper einen Gehäuseabschnitt (101) aufweist, der mindestens einen von dem Elektrodenabschnitt (40) und dem hervorstehenden Abschnitt (51) beherbergt.

6. Medizinische Vorrichtung (10, 80, 90) nach einem der Ansprüche 1 bis 5,
wobei der Schaftabschnitt (20) einen Stufenabschnitt (26) aufweist, der von einer Position innerhalb der distalen Oberfläche (25) in der radialen Richtung zu einer distalen Seite der distalen Oberfläche (25) hervorsteht.

7. Medizinische Vorrichtung (10, 80, 90) nach einem der Ansprüche 1 bis 6,
wobei die Leitung (50) mindestens ein Abschnitt einer Vielzahl von spiralförmig geflochtenen Drahtstäben (91) ist, und eine Oberfläche davon mit einem Isoliermaterial beschichtet ist.

8. Medizinische Vorrichtung (10, 80, 90) nach einem der Ansprüche 1 bis 7, ferner umfassend:
einen Ausdehnungskörper (30), der zwischen dem Schaftabschnitt (20) und dem Elektrodenabschnitt (40) angeordnet und in der radialen Richtung des Schaftabschnitts (20) nach außen aufblasbar ist.

## Revendications

1. Dispositif médical (10, 80, 90) comprenant :
une partie de tige allongée (20) ;
une pluralité de parties d'électrode (40) électriquement indépendantes disposées sur un côté distal de la partie de tige (20), s'étendant le long d'une direction longitudinale de la partie de tige (20), et déformables dans une direction radiale de la partie de tige (20) ; et
une pluralité de conducteurs (50) électriquement indépendants incluant une partie enfouie (52) enfouie dans la partie de tige (20), et permettant à un courant de circuler vers les parties d'électrode (40),
**caractérisé en ce que**
chacun de la pluralité de conducteurs (50) présente une partie saillante (51) faisant saillie depuis une surface distale (25) de la partie de tige (20) et reliée à la partie d'électrode (40) et
chacune des parties saillantes (51) est reliée à chacune des parties d'électrode (40), différentes les unes des autres,
dans lequel
chacune des parties saillantes (51) est parallèle à un axe (X) de la partie de tige (20), et au moins une partie de la partie enfouie (52) présente une forme en spirale enroulée autour de l'axe de la partie de tige.

2. Dispositif médical (10, 80, 90) selon la revendication 1, dans lequel la pluralité de parties d'électrode (40) sont disposées à égale distance dans une direction circonférentielle (Z) de la partie de tige (20).

3. Dispositif médical (10, 80, 90) selon les revendications 1 ou 2,
dans lequel lorsqu'un rayon d'un axe (X) de la partie de tige (20) vers le conducteur (50) est défini par R, le nombre des conducteurs (50) est défini par N et un angle d'inclinaison du conducteur (50) par rapport à une section transversale perpendiculaire à l'axe (X) de la partie de tige (20) est défini par θ, une distance entre des axes centraux des conducteurs (50) adjacents les uns aux autres est 2πR/N·sinθ.

4. Dispositif médical (10, 80, 90) selon la revendication 1, dans lequel la pluralité de parties d'électrode (40) sont disposées pour être sollicitées ver une partie de la partie de tige (20) dans une direction circonférentielle (Z).

5. Dispositif médical (10, 80, 90) selon l'une quelconque des revendications 1 à 4, comprenant en outre :
un corps de support annulaire disposé sur une surface périphérique externe de la partie de tige (20),
dans lequel le corps de support présente une partie de logement (101) qui loge au moins l'une de la partie d'électrode (40) et de la partie saillante (51).

6. Dispositif médical (10, 80, 90) selon l'une quelconque des revendications 1 à 5,
dans lequel la partie de tige (20) présente une partie étagée (26) faisant saillie d'une position à l'intérieur de la surface distale (25) dans la direction radiale à un côté distal de la surface distale (25).

7. Dispositif médical (10, 80, 90) selon l'une quelconque des revendications 1 à 6,
dans lequel le conducteur (50) est au moins une partie d'une pluralité de fils-machine tressés en spirale (91), et dont une surface est revêtue d'un matériau isolant.

8. Dispositif médical (10, 80, 90) selon l'une quelconque des revendications 1 à 7, comprenant en outre :
un corps d'expansion (30) situé entre la partie de tige (20) et la partie d'électrode (40), et gonflable vers l'extérieur dans la direction radiale de la partie de tige (20).
